# EUROPÄISCHE PATENTSCHRIFT

(11) **EP 4 441 189 B1**
(45) Veröffentlichungstag und Bekanntmachung des Hinweises auf die Patenterteilung: **01.10.2025**
(21) Anmeldenummer: 22801841.2
(22) Anmeldetag: 10.11.2022
(51) Int. Cl.: C11D 3/395, C11D 11/00, C07C 50/18

(54) **ANTHRACEN-9,10-DION-DERIVATE ALS PHOTOAKTIVATOREN IN WASCHMITTELN**
ANTHRACENE-9,10-DIONE DERIVATIVES AS PHOTOACTIVATORS IN DETERGENTS
DÉRIVÉS D'ANTHRACÈNE-9,10-DIONE UTILISÉS COMME PHOTOACTIVATEURS DANS DES DÉTERGENTS

(30) Priorität: 03.12.2021 DE 102021213788
(43) Veröffentlichungstag der Anmeldung: 09.10.2024
(73) Patentinhaber: Henkel AG & Co. KGaA, 40589 Düsseldorf (DE)
(72) Erfinder: KROPF, Christian, 40724 Hilden (DE); LEDERMANN, Nadia, 47053 Duisburg (DE)
(86) Internationale Anmeldenummer: PCT/EP2022/081485
(87) Internationale Veröffentlichungsnummer: WO 2023/099154

(56) Entgegenhaltungen:
- WO-A1-2004/072217
- WO-A2-2006/106049
- WILLIAM E PARHAM: "o-Benzoylbenzoic Acids the Reaction of Lithium 2-Lithiumbenzoates with Acid Chlorides. A Contribution to the Chemistry of Alizarin and Podophyllotoxin", THE JOURNAL OF ORGANIC CHEMISTRY, AMERICAN CHEMICAL SOCIETY, vol. 46, 1 January 1981 (1981-01-01), pages 1057 - 1061, XP002148658, ISSN: 0022-3263, DOI: 10.1021/JO00319A003
- DRIVAS I ET AL: "Natural anthraquinonoid colorants as platform chemicals in the synthesis of sustainable disperse dyes for polyesters", DYES AND PIGMENTS, ELSEVIER APPLIED SCIENCE PUBLISHERS BARKING, GB, vol. 88, no. 1, 1 January 2011 (2011-01-01), pages 7 - 17, XP027243207, ISSN: 0143-7208, [retrieved on 20100523]

## Beschreibung

Die vorliegende Erfindung betrifft die Verwendung von Alkyloxy-substituierten Anthracen-9,10-di-onen als Photobleichmittel zur Entfernung von Anschmutzungen von textilen Materialien, und Waschmittel, die derartige Photobleichmittel enthalten, sowie ein Verfahren zum Entfernen von Anschmutzungen von Textilmaterialien unter Einsatz Alkyloxy-substituierter Anthracen-9,10-dione in einer wässrigen Waschflüssigkeit unter Lichtbestrahlung.

Die Textilwäsche dient neben der Entfernung von Gerüchen und gegebenenfalls Beduftung der Textilien in der Regel in erster Linie der Entfernung von Anschmutzungen unterschiedlichster Natur. Diese Reinigungswirkung wird durch den Einsatz von Bleichmitteln, Tensiden und Gerüststoffen zur Waschlauge unterstützt, wobei dem Bleichmittel ein wesentlicher Anteil an der Entfernung insbesondere farbiger Anschmutzungen zukommt. Neben einer nur geringe Bedeutung zukommenden reduktiven Bleiche setzt man als Bleichmittel in der Regel Persauerstoffverbindungen ein, deren oxidative Bleichleistung herkömmlicherweise durch sogenannte Bleichaktivatoren verstärkt wird, die zum Beispiel durch Perhydrolyse von Carbonsäurederivaten wie TAED Percarbonsäuren wie Peressigsäure bilden, welche oxidationsstärker als die Ausgangs-Persauerstoffverbindung sind. Auch eine Vielzahl von Metallkomplexen sind zur Verstärkung der Bleichleistung von Persauerstoffverbindungen befähigt.

Alternativ ist vorgeschlagen worden, bleichaktive Spezies während des Waschvorgangs durch Bestrahlung von Verbindungen zu bilden, die durch die Bestrahlung in photoangeregte Zustände gelangen, welche reaktiver als der Grundzustand sind. Beispielsweise aus WO 98/32826 A1 und dem dort zitierten Stand der Technik ist bekannt, dass bestimmte wasserlösliche Phthalocyanin-, Naphthocyanin- und Metallocyanin-Verbindungen als Photobleichmittel verwendet werden können.

WO 2004/072217 A1 offenbart alkylsubstituierte Anthrachinone als Photobleichmittel in der Textilreinigung.

Ein Nachteil mancher derartiger Photobleichmittel, die synonym auch als Photoaktivatoren bezeichnet werden, ist das Erfordernis des Einwirkens hochenergetischer elektromagnetischer Strahlung wie UV-Strahlung, damit sich die bleichaktiven Spezies bilden.

Überraschenderweise wurde gefunden, dass bestimmte Anthracen-9,10-dion-Derivate dies bei Bestrahlung mit sichtbarem Licht vermögen.

Ein erster Gegenstand der Erfindung ist die Verwendung von Alkyloxy-substituierten Anthracen-9,10-dionen der allgemeinen Formel (I), 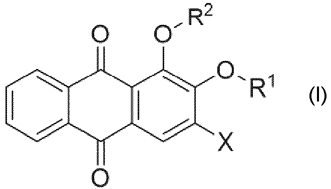 in der R¹ und R² unabhängig voneinander für H, SO₃⁻M⁺ oder einen gegebenenfalls substituierten linearen oder verzweigten Alkylrest mit 1 bis 20 C-Atomen, vorzugsweise 8 bis 16 C-Atomen stehen mit der Maßgabe, dass mindestens einer der Reste R¹ oder R² ein gegebenenfalls substituierter linearer oder verzweigter Alkylrest mit 1 bis 20 C-Atomen, vorzugsweise 8 bis 6 C-Atomen ist, X für H oder SO₃⁻M⁺ und M⁺ für ein Proton, ein Alkalimetallion oder ein Ammoniumion oder deren Mischungen stehen, als Photobleichmittel zur Entfernung von Anschmutzungen von textilen Materialien.

Bei den Anschmutzungen handelt es sich vorzugsweise um solche, die polymerisierbare Substanzen enthalten, insbesondere polymerisierbare Farbstoffe, wobei es sich bei den polymerisierbaren Farbstoffen vorzugsweise um polyphenolische Farbstoffe, insbesondere um Flavonoide, vor allem um Anthocyanidine oder Anthocyane oder Oligomere dieser Verbindungen handelt. Bei den Anschmutzungen handelt es sich ebenfalls vorzugsweise um solche, die als Farbstoffe Carotenoide und/oder Chlorophylle, also auf dem Porphyrinring beruhende Farbstoffe, enthalten. Neben der Entfernung von Anschmutzungen in den Farben grün, gelb, rot oder blau kommt auch die von Anschmutzungen in Zwischenfarben, insbesondere violett, lila, braun, purpurfarben oder rosa, und auch von Anschmutzungen in Betracht, die eine grüne, gelbe, rote, violette, lilafarbene, braune, purpurfarbene, rosafarbene oder blaue Tönung aufweisen, ohne im Wesentlichen selbst komplett aus dieser Farbe zu bestehen. Die genannten Farben können insbesondere auch jeweils hell oder dunkel sein. Es handelt sich hierbei vorzugsweise um Anschmutzungen, insbesondere um Flecken von Gras, Früchten oder Gemüse, insbesondere auch um Anschmutzungen durch Lebensmittelprodukte, wie beispielsweise Gewürze, Saucen, Chutneys, Currys, Pürees und Marmeladen, oder Getränke, wie beispielsweise Kaffee, Tee, Weine und Säfte, die entsprechende grüne, gelbe, rote, violette, lilafarbene, braune, purpurfarbene, rosafarbene und/oder blaue Farbstoffe enthalten. Die erfindungsgemäß zu entfernenden Anschmutzungen können insbesondere verursacht sein durch Kirsche, Morelle, Traube, Apfel, Granatapfel, Aronia, Pflaume, Sanddorn, Açai, Kiwi, Mango, Gras, oder Beeren, vor allem durch rote oder schwarze Johannisbeeren, Holunderbeeren, Brombeeren, Himbeeren, Blaubeeren, Preiselbeeren, Kronsbeeren, Erdbeeren oder Heidelbeeren, durch Kaffee, Tee, Rotkohl, Blutorange, Aubergine, Tomate, Karotte, Rote Beete, Spinat, Paprika, rotfleischige oder blaufleischige Kartoffel, oder rote Zwiebel.

Falls in den Verbindungen der allgemeinen Formel (I) ein Rest R¹ und/oder R² substituiert ist, trägt er vorzugsweise Substituenten ausgewählt aus -OH, -COO⁻M⁺, -SO₃⁻M⁺, -OSO₃⁻M⁺, -N⁺(CH₂CH₃)₃Hal⁻ und deren Mischungen, wobei Hal⁻ für Cl⁻, Br⁻, J⁻, F⁻ und deren Mischungen steht und M⁺ und Hal⁻ auch fehlen können, wenn -COO⁻, -SO₃⁻, -OSO₃⁻ und -N⁺(CH₂CH₃)₃ in ladungsausgleichender Menge vorhanden sind; auch bevorzugt ist, wenn mindestens 1 Substituent endständig ist.

Die genannten Alkyloxy-substituierten Anthracen-9,10-dione können als solche oder in Form von Waschmitteln, welche sie und gegebenenfalls weitere konventionelle Waschmittelinhaltstoffe enthalten, erfindungsgemäß verwendet werden.

Ein zweiter Gegenstand der Erfindung ist daher ein Waschmittel, enthaltend ein nichtionisches, anionisches, kationisches und/oder amphoteres Tensid sowie ein oben definiertes Alkyloxy-substituierten Anthracen-9,10-dion. Vorzugsweise enthält das Waschmittel 0,00001 Gew.-% bis 1 Gew.-%, insbesondere 0,001 Gew.-% bis 0,1 Gew.-% Alkyloxy-substituiertes Anthracen-9,10-dion.

Ein weiterer Gegenstand ist ein Verfahren zum Entfernen von Anschmutzungen von Textilmaterialien unter Einsatz der oben definierten Alkyloxy-substituierten Anthracen-9,10-dione in einer wässrigen Waschflüssigkeit, in welcher sich die waschbedürftigen angeschmutzten Textilmaterialien befinden, unter Bestrahlung der wässrigen Waschflüssigkeit mit sichtbarem Licht.

Unter sichtbarem Licht soll hier für das menschliche Auge sichtbare elektromagnetische Strahlung verstanden werden, deren Wellenlänge im Bereich von 400 nm bis 780 nm liegt. Im Rahmen der vorliegenden Erfindung kommt vorzugsweise Licht im Wellenlängenbereich von 400 nm bis 600 nm, insbesondere von 450 nm bis 525 nm, zum Einsatz. Dabei kann es sich um Tages- beziehungsweise Sonnenlicht handeln, oder um künstlich erzeugtes Licht, wobei letzteres vorzugsweise mit Hilfe eines LED-Leuchtkörpers erzeugt wird. Im Rahmen des erfindungsgemäßen Verfahrens wirkt das Licht auf die Waschflüssigkeit ein, in welcher sich die waschbedürftigen angeschmutzten Textilmaterialien und das Alkyloxy-substituierte Anthracen-9,10-dion befinden. Dies kann beispielsweise geschehen, indem man den Waschvorgang manuell in einem offenen Behälter ausführt, dessen Öffnung dem natürlichen Tages- oder Sonnenlicht ausgesetzt ist. Auch möglich ist der Einsatz einer herkömmlichen Waschmaschine, wenn diese ein sogenanntes Bullauge aufweist, durch welches Licht ins Innere der Maschine und zur darin befindlichen Waschflüssigkeit gelangt. Der Einsatz alternativer Lichtquellen, zum Beispiel von LEDs, ist auch möglich, wobei die Lichtquelle bei Einsatz einer Waschmaschine vorzugsweise im Inneren der Maschine derart angeordnet ist, dass sie die Waschflüssigkeit wenigstens zeitweilig, zum Beispiel bei deren Umpumpen, bestrahlt. Bei der letztgenannten Variante ist es auch möglich, die Lichtquelle nicht über den gesamten Zeitraum des Waschvorgangs zu betreiben, sondern nur für einen Teil desselben, so dass das oxidative Bleichen nur während dieses Teilzeitraums stattfindet.

Im Rahmen der erfindungsgemäßen Verwendung und der erfindungsgemäßen Verfahrens ist es bevorzugt, das genannte Alkyloxy-substituierte Anthracen-9,10-dion in Konzentrationen im Bereich von 0,0001 mmol/I bis 0,1 mmol/l, insbesondere von 0,001mmol/l bis 0,01 mmol/I in wässrigen Waschlaugen einzusetzen.

Das Waschmittel kann in jeder nach dem Stand der Technik etablierten und/oder jeder zweckmäßigen Darreichungsform vorliegen. Dazu zählen beispielsweise feste, pulverförmige, flüssige, gelförmige oder pastöse Darreichungsformen, gegebenenfalls auch aus mehreren Phasen bestehend; ferner gehören beispielsweise dazu: Extrudate, Granulate, Tabletten oder Pouches, sowohl in Großgebinden als auch portionsweise abgepackt. In einer bevorzugten Ausführungsform ist das Mittel flüssig.

Das erfindungsgemäße Verfahren wird ausgeführt und die erfindungsgemäße Verwendung erfolgt in einer jeweils bevorzugten Ausführungsform durch den Einsatz eines erfindungsgemäßen Wasch-und Reinigungsmittels, das keine Bleichmittel enthält. Hierunter ist zu verstehen, dass das Mittel keine Bleichmittel im engeren Sinne, also Hypochlorite oder Persauerstoffverbindungen, enthält. Bei dem Waschmittel handelt es sich in einer besonders bevorzugten Ausführungsform um ein flüssiges Textilwaschmittel.

Erfindungsgemäße Waschmittel können über das erfindungswesentliche Alkyloxy-substituierte Anthracen-9,10-dion und Tensid hinaus übliche sonstige Bestandteile von Textilwaschmitteln enthalten, insbesondere ausgewählt aus der Gruppe der Gerüststoffe, Polymere, Enzyme, Duftstoffe und Parfümträger.

Zu den Gerüststoffe zählen insbesondere die Zeolithe, Silikate, Carbonate, organische Cobuilder und - sofern keine ökologischen Bedenken gegen ihren Einsatz bestehen - auch die Phosphate.

Der feinkristalline, synthetische und gebundenes Wasser enthaltende Zeolith ist vorzugsweise Zeolith A und/oder Zeolith P. Als Zeolith P kommt beispielsweise Zeolith MAP^{®} (Handelsprodukt der Firma Crosfield) in Frage. Geeignet sind jedoch auch Zeolith X sowie Mischungen aus Zeolith A, X und/oder P. Kommerziell erhältlich und im Rahmen der vorliegenden Erfindung einsetzbar ist beispielsweise auch ein Co-Kristallisat aus Zeolith X und Zeolith A (ca. 80 Gew.-% Zeolith X), das durch die Formel

n Na₂O · (1-n) K₂O · Al₂O₃ · (2 - 2,5) SiO₂ · (3,5 - 5,5) H₂O

beschrieben werden kann. Der Zeolith kann dabei sowohl als Gerüststoff in einem granularen Compound eingesetzt, als auch zu einer Art "Abpuderung" einer granularen Mischung, vorzugsweise einer zu verpressenden Mischung verwendet werden, wobei üblicherweise beide Wege zur Inkorporation des Zeoliths in das Vorgemisch genutzt werden. Zeolithe können eine mittlere Teilchengröße von weniger als 10 µm (Volumenverteilung; Meßmethode: Coulter Counter) aufweisen und enthalten vorzugsweise 18 Gew.-% bis 22 Gew.-%, insbesondere 20 Gew.-% bis 22 Gew.-% an gebundenem Wasser.

Es können auch kristalline schichtförmige Silikate der allgemeinen Formel NaMSiₓO2ₓ₊₁ · y H₂O eingesetzt werden, worin M Natrium oder Wasserstoff darstellt, x eine Zahl von 1,9 bis 22, vorzugsweise von 1,9 bis 4 ist, wobei besonders bevorzugte Werte für x 2, 3 oder 4 sind, und y für eine Zahl von 0 bis 33, vorzugsweise von 0 bis 20 steht. Die kristallinen schichtförmigen Silikate der Formel NaMSiₓO2ₓ₊₁ · y H₂O werden beispielsweise von der Firma Clariant GmbH (Deutschland) unter dem Handelsnamen Na-SKS vertrieben. Beispiele für diese Silikate sind Na-SKS-1 (Na₂Si₂₂O₄₅ · x H₂O, Kenyait), Na-SKS-2 (Na₂Si₁₄O₂₉ · x H₂O, Magadiit), Na-SKS-3 (Na₂Si₈O₁₇ · x H₂O) oder Na-SKS-4 (Na2Si4O9 · x H2O, Makatit).

Bevorzugt sind kristalline Schichtsilikate der Formel NaMSiₓO2ₓ₊₁ · y H₂O, in denen x für 2 steht. Insbesondere sind sowohl ß- als auch δ-Natriumdisilikate Na₂Si₂O₅ · y H₂O sowie weiterhin vor allem Na-SKS-5 (α-Na₂Si₂O₅), Na-SKS-7 (ß-Na₂Si₂O₅, Natrosilit), Na-SKS-9 (NaHSi₂O₅ · H₂O), Na-SKS-10 (NaHSi₂O₅ · 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅) bevorzugt. Waschmittel enthalten vorzugsweise einen Gewichtsanteil des kristallinen schichtförmigen Silikats der Formel NaMSiₓO2ₓ₊₁ · y H₂O von 0,1 Gew.-% bis 20 Gew.-%, bevorzugt von 0,2 Gew.-% bis 15 Gew.-% und insbesondere von 0,4 Gew.-% bis 10 Gew.-%.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Unter dem Begriff "amorph" wird verstanden, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen, hervorrufen.

Alternativ oder in Kombination mit den vorgenannten amorphen Natriumsilikaten können röntgenamorphe Silikate eingesetzt werden, deren Silikatpartikel bei Elektronenbeugungsexperimenten verwaschene oder sogar scharfe Beugungsmaxima liefern. Dies ist so zu interpretieren, dass die Produkte mikrokristalline Bereiche der Größe zehn bis einige Hundert nm aufweisen, wobei Werte bis max. 50 nm und insbesondere bis max. 20 nm bevorzugt sind. Derartige röntgenamorphe Silikate weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Diese(s) Silikat(e), vorzugsweise Alkalisilikate, besonders bevorzugt kristalline oder amorphe Alkalidisilikate, sind, wenn vorhanden, in Waschmitteln in Mengen von 3 Gew.-% bis 60 Gew.-%, vorzugsweise von 8 Gew.-% bis 50 Gew.-% und insbesondere von 20 Gew.-% bis 40 Gew.-% enthalten.

Auch ein Einsatz der allgemein bekannten Phosphate als Buildersubstanzen ist möglich, sofern ein derartiger Einsatz nicht aus ökologischen Gründen vermieden werden soll. Unter der Vielzahl der kommerziell erhältlichen Phosphate haben die Alkalimetallphosphate unter besonderer Bevorzugung von Pentanatrium- und Pentakaliumtriphosphat (Natrium- und Kaliumtripolyphosphat) in der Wasch- und Reinigungsmittelindustrie die größte Bedeutung. Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei. Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat). Bevorzugt eingesetzt werden weiterhin die Natriumkaliumtripolyphosphate. Werden Phosphate in Waschmitteln eingesetzt, so enthalten bevorzugte Mittel diese(s) Phosphat(e), vorzugsweise Alkalimetallphosphat(e), besonders bevorzugt Pentanatrium- bzw. Pentakaliumtriphosphat (Natrium- bzw. Kaliumtripolyphosphat), in Mengen von 5 Gew.-% bis 80 Gew.-%, vorzugsweise von 15 Gew.-% bis 75 Gew.-% und insbesondere von 20 Gew.-% bis 70 Gew.-%.

Weiter einsetzbar sind Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei bevorzugt die Alkalicarbonate, insbesondere Natriumcarbonat, Natriumhydrogencarbonat oder Natriumsesquicarbonat eingesetzt werden. Besonders bevorzugt kann ein Buildersystem enthaltend eine Mischung aus Tripolyphosphat und Natriumcarbonat sein. Aufgrund ihrer im Vergleich mit anderen Buildersubstanzen geringen chemischen Kompatibilität mit den übrigen Inhaltsstoffen von Waschmitteln werden die Alkalimetallhydroxide üblicherweise nur in geringen Mengen, vorzugsweise in Mengen unterhalb 10 Gew.-%, bevorzugt unterhalb 6 Gew.-%, besonders bevorzugt unterhalb 4 Gew.-% und insbesondere unterhalb 2 Gew.-%, eingesetzt. Besonders bevorzugt werden Mittel, welche bezogen auf ihr Gesamtgewicht weniger als 0,5 Gew.-% und insbesondere keine Alkalimetallhydroxide enthalten. Bevorzugt ist der Einsatz von Carbonat(en) und/oder Hydrogencarbonat(en), vorzugsweise Alkalicarbonat(en), besonders bevorzugt Natriumcarbonat, in Mengen von 2 Gew.-% bis 50 Gew.-%, vorzugsweise von 5 Gew.-% bis 40 Gew.-% und insbesondere von 7,5 Gew.-% bis 30 Gew.-%.

Als organische Builder sind insbesondere Polycarboxylate/Polycarbonsäuren, polymere Polycarboxylate, Asparaginsäure, Polyacetale, Dextrine sowie Phosphonate zu nennen. Brauchbar sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Waschmitteln. Insbesondere sind hierbei Citronensäure, Bernsteinsäure, Glutarsäure, Adipinsäure, Gluconsäure und beliebige Mischungen aus diesen zu nennen. Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 g/mol bis 70000 g/mol. Geeignet sind insbesondere Polyacrylate, die bevorzugt eine Molekülmasse von 2000 g/mol bis 20000 g/mol aufweisen. Aufgrund ihrer überlegenen Löslichkeit können aus dieser Gruppe wiederum die kurzkettigen Polyacrylate, die Molmassen von 2000 g/mol bis 10000 g/mol, und besonders bevorzugt von 3000 g/mol bis 5000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 Gew.-% bis 90 Gew.-% Acrylsäure und 50 Gew.-% bis 10 Gew.-% Maleinsäure enthalten. Ihre relative Molekülmasse, bezogen auf freie Säuren, beträgt im Allgemeinen 2000 g/mol bis 70000 g/mol, vorzugsweise 20000 g/mol bis 50000 g/mol und insbesondere 30000 g/mol bis 40000 g/mol. Zur Verbesserung der Wasserlöslichkeit können die Polymere auch Allylsulfonsäuren, wie beispielsweise Allyloxybenzolsulfonsäure und Methallylsulfonsäure, als Monomer enthalten. Die (co-)polymeren Polycarboxylate können als Feststoff oder in wässriger Lösung eingesetzt werden. Der Gehalt von Waschmitteln an (co-)polymeren Polycarboxylaten beträgt vorzugsweise 0,5 Gew.-% bis 20 Gew.-% und insbesondere 3 Gew.-% bis 10 Gew.-%.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten. Weitere bevorzugte Copolymere sind solche, die als Monomere Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren und/oder deren Salze.

Eine weitere Substanzklasse mit Buildereigenschaften stellen die Phosphonate dar. Dabei handelt es sich um die Salze von insbesondere Hydroxyalkan- oder Aminoalkanphosphonsäuren. Unter den Hydroxyalkanphosphonsäuren ist die 1-Hydroxyethan-1,1-diphosphonsäure (HEDP) von besonderer Bedeutung. Sie wird insbesondere als Natriumsalz eingesetzt, wobei das Dinatriumsalz neutral und das Tetranatriumsalz alkalisch reagiert. Als Aminoalkanphosphonsäuren kommen insbesondere Ethylendiamintetramethylenphosphonsäure (EDTMP), Diethylentriaminpenta-methylenphosphonsäure (DTPMP) sowie deren höhere Homologe in Frage. Sie werden insbesondere in Form der neutral reagierenden Natriumsalze, so zum Beispiel als Hexanatriumsalz der EDTMP oder als Heptaund Octa-Natriumsalz der DTPMP, eingesetzt. Auch Mischungen aus den genannten Phosphonaten können als organische Builder verwendet werden. Insbesondere die Aminoalkanphosphonate besitzen zudem ein ausgeprägtes Schwermetallbindevermögen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 g/mol bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 g/mol bis 30000 g/mol. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Gewünschtenfalls geeignete Einsatzmengen liegen insbesondere in zeolithhaltigen und/oder silicathaltigen Formulierungen bei 3 Gew.-% bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Darüber hinaus können alle Verbindungen, die in der Lage sind, Komplexe mit Erdalkaliionen auszubilden, als Gerüststoffe eingesetzt werden.

Wasch- und Reinigungsmittel gemäß der Erfindung müssen nichtionische, anionische, kationische und/oder amphotere Tenside enthalten. Ein erfindungsgemäßes Waschmittel enthält vorzugsweise nichtionisches und/oder anionisches Tensid

Als nichtionische Tenside können alle dem Fachmann bekannten nichtionischen Tenside eingesetzt werden. Mit besonderem Vorzug enthalten Waschmittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vor-zugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C12-14-Alkohole mit 3 EO oder 4 EO, C9-11-Alkohol mit 7 EO, C13-15-Alkohole mit 3 EO, 5 EO, 7 EO oder 8 EO, C12-18-Alkohole mit 3 EO, 5 EO oder 7 EO und Mischungen aus diesen, wie Mischungen aus C12-14-Alkohol mit 3 EO und C12-18-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE).

Alternativ oder zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Außerdem können als weitere nichtionische Tenside auch Alkylglykoside der allgemeinen Formel RO(G)x eingesetzt werden, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Mono-glykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können eingesetzt werden. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel, 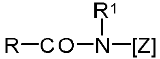 in der R für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgender Acylierung mit einer Fettsäure, einem Fettsäurealkylester oder einem Fettsäurechlorid erhalten werden können. Zur Gruppe der Polyhydroxyfettsäureamide gehören auch Verbindungen der Formel 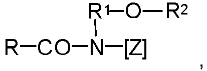 in der R für einen linearen oder verzweigten Alkyl- oder Alkenylrest mit 7 bis 12 Kohlenstoffatomen, R¹ für einen linearen, verzweigten oder zyklischen Alkylrest oder einen Arylrest mit 2 bis 8 Kohlenstoffatomen und R² für einen linearen, verzweigten oder zyklischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden.

In Reinigungsmitteln sind Niotenside aus der Gruppe der alkoxylierten Alkohole, besonders bevorzugt aus der Gruppe der gemischt alkoxylierten Alkohole und insbesondere aus der Gruppe der EO/AO/EO-Niotenside, oder der PO/AO/PO-Niotenside, speziell der PO/EO/PO-Niotenside besonders bevorzugt. Solche PO/EO/PO-Niotenside zeichnen sich durch gute Schaumkontrolle aus.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 Gew.-% bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen, darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

An Stelle der genannten Tenside oder in Verbindung mit ihnen können auch kationische und/oder amphotere Tenside eingesetzt werden.

Als kationische Aktivsubstanzen können beispielsweise kationische Verbindungen der nachfolgenden Formeln eingesetzt werden: 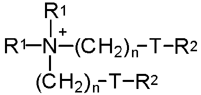 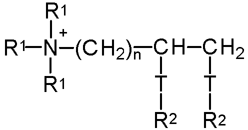 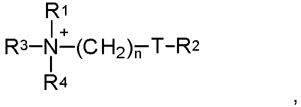 worin jede Gruppe R¹ unabhängig voneinander ausgewählt ist aus C₁₋₆-Alkyl-, -Alkenyloder -Hydroxyalkylgruppen; jede Gruppe R² unabhängig voneinander ausgewählt ist aus C₈₋₂₈-Alkyloder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist.

Zur Pflege der Textilien und zur Verbesserung der Textileigenschaften wie einem weicheren "Griff" (Avivage) und verringerter elektrostatischer Aufladung (erhöhter Tragekomfort) können textilweichmachende Verbindungen eingesetzt werden. Die Wirkstoffe dieser Formulierungen sind quartäre Ammoniumverbindungen mit zwei hydrophoben Resten, wie beispielsweise das Disteraryldimethylammoniumchlorid, welches jedoch wegen seiner ungenügenden biologischen Abbaubarkeit zunehmend durch quartäre Ammoniumverbindungen ersetzt wird, die in ihren hydrophoben Resten Estergruppen als Sollbruchstellen für den biologischen Abbau enthalten.

Derartige "Esterquats" mit verbesserter biologischer Abbaubarkeit sind beispielsweise dadurch erhältlich, dass man Mischungen von Methyldiethanolamin und/oder Triethanolamin mit Fettsäuren verestert und die Reaktionsprodukte anschließend in an sich bekannter Weise mit Alkylierungsmitteln quaterniert. Als Appretur weiterhin geeignet ist Dimethylolethylenharnstoff.

Zur Steigerung der Leistung von Waschmitteln sind Enzyme einsetzbar. Hierzu gehören insbesondere Proteasen, Amylasen, Lipasen, Hemicellulasen, Cellulasen, Perhydrolasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Waschmittel enthalten Enzyme vorzugsweise in Gesamtmengen von 1 x 10⁻⁶ Gew.-% bis 5 Gew.-% bezogen auf aktives Protein. Die Proteinkonzentration kann mit Hilfe bekannter Methoden, zum Beispiel dem BCA-Verfahren oder dem Biuret-Verfahren bestimmt werden.

Unter den Proteasen sind solche vom Subtilisin-Typ bevorzugt. Beispiele hierfür sind die Subtilisine BPN' und Carlsberg sowie deren weiterentwickelte Formen, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7.

Beispiele für einsetzbare Amylasen sind die α-Amylasen aus Bacillus licheniformis, aus B. amyloliquefaciens, aus B. stearothermophilus, aus Aspergillus niger und A. oryzae sowie die für den Einsatz in Wasch- und Reinigungsmitteln verbesserten Weiterentwicklungen der vorgenannten Amylasen. Des Weiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben.

Einsetzbar sind wegen ihrer Triglycerid-spaltenden Aktivität Lipasen oder Cutinasen. Hierzu gehören beispielsweise die ursprünglich aus Humicola lanuginosa (Thermomyces lanuginosus) erhältlichen oder aus diesen weiterentwickelten Lipasen, insbesondere solche mit dem Aminosäureaustausch D96L. Des Weiteren sind beispielsweise die Cutinasen einsetzbar, die ursprünglich aus Fusarium solani pisi und Humicola insolens isoliert worden sind. Einsetzbar sind weiterhin Lipasen und/oder Cutinasen, deren Ausgangsenzyme ursprünglich aus Pseudomonas mendocina und Fusarium solanii isoliert worden sind.

Weiterhin können Enzyme eingesetzt werden, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen.

Zur Erhöhung der bleichenden Wirkung können gewünschtenfalls Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Die Enzyme können in jeder nach dem Stand der Technik etablierten Form eingesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt. Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil. Weiterhin ist es möglich, zwei oder mehrere Enzyme zusammen zu konfektionieren, so dass ein einzelnes Granulat mehrere Enzymaktivitäten aufweist.

Bevorzugt werden ein oder mehrere Enzyme und/oder Enzymzubereitungen, vorzugsweise Protease-Zubereitungen und/oder Amylase-Zubereitungen, in Mengen von 0,1 Gew.-% bis 5 Gew.-%, vorzugsweise von 0,2 Gew.-% bis 4,5 Gew.-% und insbesondere von 0,4 Gew.-% bis 4 Gew.-%, eingesetzt.

Als Parfümöle bzw. Duftstoffe können einzelne Riechstoffverbindungen, z.B. synthetische Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pinien-, Citrus-, Jasmin-, Patchouly, Rosen- oder Ylang-Ylang-Öl. Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 g/mol, während Molmassen von 300 g/mol und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note oder body) sowie "Basisnote" (end note oder dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt. Die Duftstoffe können direkt verarbeitet werden, es kann aber auch vorteilhaft sein, die Duftstoffe auf Träger aufzubringen, die durch eine langsamere Duftfreisetzung für langanhaltenden Duft sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

Bei der Wahl des Färbemittels muss beachtet werden, dass die Färbemittel eine hohe Lagerstabilität und Unempfindlichkeit gegenüber Licht sowie keine zu starke Affinität gegenüber textilen Oberflächen und hier insbesondere gegenüber Kunstfasern aufweisen können. Gleichzeitig ist auch zu berücksichtigen, dass Färbemittel unterschiedliche Stabilitäten gegenüber der Oxidation aufweisen können. Im allgemeinen gilt, dass wasserunlösliche Färbemittel gegen Oxidation stabiler sind als wasserlösliche Färbemittel. Abhängig von der Löslichkeit und damit auch von der Oxidationsempfindlichkeit variiert die Konzentration des Färbemittels in den Waschmitteln. Bei gut wasserlöslichen Färbemitteln werden typischerweise Färbemittel-Konzentrationen im Bereich von einigen 10⁻² Gew.-% bis 10⁻³ Gew.-% gewählt. Bei den auf Grund ihrer Brillanz insbesondere bevorzugten, allerdings weniger gut wasserlöslichen Pigmentfarbstoffen liegt die geeignete Konzentration des Färbemittels in Waschmitteln dagegen typischerweise bei einigen 10⁻³ Gew.-% bis 10⁻⁴ Gew.-%. Es werden Färbemittel bevorzugt, die im Waschprozess oxidativ zerstört werden können sowie Mischungen derselben mit geeigneten blauen Farbstoffen, sogenannten Blautönern. Es hat sich als vorteilhaft erwiesen, Färbemittel einzusetzen, die in Wasser oder bei Raumtemperatur in flüssigen organischen Substanzen löslich sind. Geeignet sind beispielsweise anionische Färbemittel, zum Beispiel anionische Nitrosofarbstoffe.

Zusätzlich zu den bisher genannten Komponenten können die Waschmittel weitere Inhaltsstoffe enthalten, welche die anwendungstechnischen und/oder ästhetischen Eigenschaften dieser Mittel weiter verbessern. Bevorzugte Mittel enthalten einen oder mehrere Stoffe aus der Gruppe der Elektrolyte, pH-Stellmittel, Fluoreszenzmittel, Hydrotope, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffen, Germizide, Fungizide, Antioxidantien, Antistatika, Bügelhilfsmittel, Phobier- und Imprägniermittel, Quell- und Schiebefestmittel sowie UV-Absorber.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Waschmitteln bevorzugt.

Um den pH-Wert von Waschmitteln in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 1 Gew.-% der Gesamtformulierung nicht.

Als Schauminhibitoren, kommen Seifen, Öle, Fette, Paraffine oder Silikonöle in Betracht, die gegebenenfalls auf Trägermaterialien aufgebracht sein können. Als Trägermaterialien eignen sich beispielsweise anorganische Salze wie Carbonate oder Sulfate, Cellulosederivate oder Silikate sowie Mischungen der vorgenannten Materialien. Im Rahmen der vorliegenden Anmeldung bevorzugte Mittel enthalten Paraffine, vorzugsweise unverzweigte Paraffine (n-Paraffine) und/oder Silikone, vorzugsweise linear-polymere Silikone, welche nach dem Schema (R₂SiO)ₓ aufgebaut sind und auch als Silikonöle bezeichnet werden. Diese Silikonöle stellen gewöhnlich klare, farblose, neutrale, geruchsfreie, hydrophobe Flüssigkeiten mit einem Molekulargewicht zwischen 1000 g/mol und 150000 g/mol und Viskositäten zwischen 10 mPa·s und 1000000 mPa·s dar.

Geeignete Antiredepositionsmittel sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether.

Als soil repellents kommen die aus dem Stand der Technik bekannten Polymere der Phthalsäure und/oder Terephthalsäure bzw. deren Derivate, insbesondere Polymere aus Ethylenterephthalat und/oder Polyethylenglycolterephthalat oder anionisch und/oder nichtionisch modifizierten Derivaten von diesen. Insbesondere bevorzugt von diesen sind die sulfonierten Derivate der Phthalsäure- und Terephthalsäure-Polymere.

Optische Aufheller können insbesondere den Waschmitteln zugesetzt werden, um Vergrauungen und Vergilbungen der behandelten Textilien zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate verwenden, zum Beispiel abgebaute Stärke und/oder Aldehydstärken. Auch Polyvinylpyrrolidon ist brauchbar. Als Vergrauungsinhibitoren einsetzbar sind weiterhin Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxy-methylcellulose und deren Gemische.

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können synthetische Knitterschutzmittel eingesetzt werden. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Phobier- und Imprägnierverfahren dienen der Ausrüstung von Textilien mit Substanzen, welche die Ablagerung von Schmutz verhindern oder dessen Auswaschbarkeit erleichtern. Bevorzugte Phobierund Imprägniermittel sind perfluorierte Fettsäuren, auch in Form ihrer Aluminium- u. Zirkoniumsalze, organische Silikate, Silikone, Polyacrylsäureester mit perfluorierter Alkohol-Komponente oder mit perfluoriertem Acyl- oder Sulfonyl-Rest gekoppelte, polymerisierbare Verbindungen. Auch Antistatika können enthalten sein. Die schmutzabweisende Ausrüstung mit Phobier- und Imprägniermitteln wird oft als eine Pflegeleicht-Ausrüstung eingestuft. Das Eindringen der Imprägniermittel in Form von Lösungen oder Emulsionen der betreffenden Wirkstoffe kann durch Zugabe von Netzmitteln erleichtert werden, welche die Oberflächenspannung herabsetzen. Ein weiteres Einsatzgebiet von Phobierund Imprägniermitteln ist die wasserabweisende Ausrüstung von Textilwaren, Zelten, Planen, Leder usw., bei der im Gegensatz zum Wasserdichtmachen die Gewebeporen nicht verschlossen werden, der Stoff also atmungsaktiv bleibt (Hydrophobieren). Die zum Hydrophobieren verwendeten Hydrophobiermittel überziehen Textilien, Leder, Papier, Holz usw. mit einer sehr dünnen Schicht hydrophober Gruppen, wie längere Alkyl-Ketten oder Siloxan-Gruppen. Geeignete Hydrophobiermittel sind zum Beispiel Paraffine, Wachse, Metallseifen usw. mit Zusätzen an Aluminium- oder Zirkonium-Salzen, quartäre Ammonium-Verbindungen mit langkettigen Alkyl-Resten, Harnstoff-Derivate, Fettsäure-modifizierte Melaminharze, Chrom-Komplexsalze, Silikone, Zinn-organische Verbindungen und Glutardialdehyd sowie perfluorierte Verbindungen. Die hydrophobierten Materialien fühlen sich nicht fettig an; dennoch perlen - ähnlich wie an gefetteten Stoffen - Wassertropfen an ihnen ab, ohne zu benetzen. So haben z.B. Silikon-imprägnierte Textilien einen weichen Griff und sind wasser- und schmutzabweisend; Flecke aus Tinte, Wein, Fruchtsäften und dergleichen sind leichter zu entfernen.

Zur Bekämpfung von Mikroorganismen können antimikrobielle Wirkstoffe eingesetzt werden. Hierbei unterscheidet man je nach antimikrobiellem Spektrum und Wirkungsmechanismus zwischen Bakteriostatika und Bakteriziden, Fungistatika und Fungiziden. Stoffe aus diesen Gruppen sind beispielsweise Benzalkoniumchloride, Alkylarylsulfonate, Halogenphenole und Phenolmercuriacetat, wobei auch gänzlich auf diese Verbindungen verzichtet werden kann.

Um unerwünschte, durch Luftsauerstoffeinwirkung und andere oxidative Prozesse verursachte Veränderungen an den Wasch- und Reinigungsmitteln und/oder den behandelten Textilien zu verhindern, können die Mittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite und Phosphonate.

Ein erhöhter Tragekomfort kann aus der zusätzlichen Verwendung von Antistatika resultieren. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (oder Stearyl-) dimethylbenzylammoniumchloride eignen sich ebenfalls als Antistatika für Textilien bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten Textilien und zur Erleichterung des Bügelns der behandelten Textilien können in Textilwaschmitteln Silikonderivate eingesetzt werden. Diese verbessern zusätzlich das Ausspülverhalten von Waschmitteln durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Weitere bevorzugte Silikone sind die Polyalkylenoxid-modifizierten Polysiloxane, also Polysiloxane, welche beispielsweise Polyethylenglykole aufweisen, sowie die Polyalkylenoxid-modifizierten Dimethylpolysiloxane.

Schließlich können auch UV-Absorber eingesetzt werden, die auf die behandelten Textilien aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

Proteinhydrolysate sind auf Grund ihrer faserpflegenden Wirkung weitere geeignete Aktivsubstanzen. Proteinhydrolysate sind Produktgemische, die durch sauer, basisch oder enzymatisch katalysierten Abbau von Proteinen (Eiweißen) erhalten werden. Proteinhydrolysate sowohl pflanzlichen als auch tierischen Ursprungs können eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Bevorzugt ist die Verwendung von Proteinhydrolysaten pflanzlichen Ursprungs, z.B. Soja-, Mandel-, Reis-, Erbsen-, Kartoffel- und Weizenproteinhydrolysate. Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyroglutaminsäure eingesetzt werden. Ebenfalls möglich ist der Einsatz von Derivaten der Proteinhydrolysate, beispielsweise in Form ihrer Fettsäure-Kondensationsprodukte.

Die Waschmittel können als Formkörper vorliegen. Um den Zerfall solcher vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln oder Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.-%, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Die hierin beschriebenen Waschmittel können in Dosiereinheiten vorkonfektioniert vorliegen. Diese Dosiereinheiten umfassen vorzugsweise die für einen Waschgang notwendige Menge des Mittels. Bevorzugte Dosiereinheiten weisen ein Gewicht zwischen 10 g und 50 g, bevorzugt zwischen 15 g und 40 g auf. Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Waschmaschine gewährleistet ist. Das Volumen der Dosiereinheit beträgt daher bevorzugt zwischen 10 und 35 ml, vorzugsweise zwischen 12 und 30 ml.

Die Waschmittel, insbesondere die vorgefertigten Dosiereinheiten, weisen mit besonderem Vorzug eine wasserlösliche Umhüllung auf. Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Die wasserlösliche Verpackung kann eine oder mehr Kammern aufweisen. Das Mittel kann in einer oder mehreren Kammern, falls vorhanden, der wasserlöslichen Umhüllung enthalten sein.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf. Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10 000 g/mol bis 1 000 000 g/mol, vorzugsweise von 20 000 g/mol bis 500000 g/mol, besonders bevorzugt von 30 000 g/mol bis 100000 g/mol und insbesondere von 40 000 g/mol bis 80 000 g/mol liegt. Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 Mol-% bis 100 Mol-%, vorzugsweise 80 Mol-% bis 90 Mol-%, besonders bevorzugt 81 Mol-% bis 89 Mol-% und insbesondere 82 Mol-% bis 88 Mol-% ausmacht. Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren. Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist. Ebenfalls bevorzugte Polyvinylalkoholcopolymere umfassen neben Vinylalkohol eine ethylenisch ungesättigte Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus. Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus. Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

### Beispiele

### Beispiel 1: Herstellung substituierter Anthracendione

### a) Synthese von 1,2-bis(octyloxy)anthracen-9,10-dion

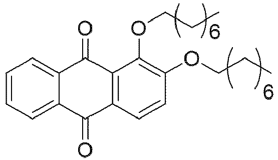

In Anlehnung an veröffentlichte Verfahren (X. Chen, K. Ding, L. Jun, Synthesis, identification and application of aldehyde reactive dyes, Dyes and Pigments 2015, 123, 404-412 und loannis Drivas, Richard S. Blackburn, Christopher M. Rayner, Natural anthraquinonoid colorants as platform chemicals in the synthesis of sustainable disperse dyes for polyesters, Dyes and Pigments 2011, 88, 7-17) wurde in einem Rundkolben ein Gemisch aus 30 ml Dimethylacetamid, 2,00 g Alizarin (8,30 mmol) und 3,50 g Kaliumcarbonat (25 mmol) auf 80° C erhitzt und für 30 min miteinander umgesetzt. Anschließend wurden 4,80 g 1-Bromoctan (25 mmol) dazugegeben und das Reaktionsgemisch wurde weitere 16 h gerührt. Zum Abbruch der Reaktion wurde das Gemisch auf Raumtemperatur gekühlt und mit Dimethylacetamid versetzt. Das Gemisch wurde filtriert und der Rückstand so lange mit Dimethylacetamid gewaschen, bis ein gelber Feststoff zu erkennen war. Der Rückstand wurde anschließend unter vermindertem Druck getrocknet. Man erhielt 1,2-Bis(octyloxy)anthracen-9,10-dion (M1) als gelben Feststoff mit einer Ausbeute von 94%.

### b) Herstellung weiterer 1,2-bis(alkyloxy)anthracen-9,10-dione

Analog zum in a) beschriebenen Vorgehen wurden 1,2-Bis(decyloxy)anthracen-9,10-dion (M2), 1,2-Bis(dodecyloxy)anthracen-9,10-dion (M3) und 1,2-Bis(hexadecyloxy)anthracen-9,10-dion (M4) hergestellt.

### Beispiel 2: Abbau von Lycopin

Anhand einer vereinfachten Modellreaktion wurde die Entfärbung des roten, aus der Tomate stammenden Farbstoffs Lycopin anhand eines UV/VIS-Spektrums visualisiert und charakterisiert. Dazu wurden gleiche Volumenteile einer Lösung aus 0,01 mM Lycopin in Chloroform und einer Lösung aus 0,0001 mM jeweils einer in Beispiel 1 hergestellten Verbindung in Chloroform in einer Küvette gemischt und die Küvette in ein Spektrometer Specord S600, Analytik Jena, gestellt; Meßwellenläge 480 nm. Mit einem möglichst kleinen Abstand zwischen der Küvette und einer blau leuchtenden LED-Lampe (450 nm) wurde die Kinetik des Abbaus des Farbstoffs Lycopin bestimmt. Es ergaben sich die in der nachfolgenden Tabelle 1 angeführten Daten.

**Tabelle 1: Abbau von Lycopin, Reaktion Pseudo 1. Ordnung**

| Photoaktivator | K [min⁻¹] | t_{1/2} [min] | Absorptionsmaximum (t = 0 min) [a.u.] | Absorptionsmaximum (t = 5 min) [a.u.] | Abbau [%] |
|---|---|---|---|---|---|
| - | 0,006 | 107 | 1,00 | 0,98 | 2 |
| M1 | 0,28 | 2,5 | 1,00 | 0,40 | 60 |
| M2 | 0,28 | 2,5 | 1,00 | 0,47 | 53 |
| M3 | 0,28 | 2,5 | 1,00 | 0,51 | 49 |
| M4 | 0,28 | 2,5 | 1,00 | 0,53 | 47 |

### Beispiel 3: Abbau von β-Carotin

Beispiel 2 wurde wiederholt unter Einsatz des gelben, unter anderem aus der Karotte stammenden Farbstoffs β-Carotin an Stelle von Lycopin und einer Messwellenlänge von 463 nm. Es ergaben sich die in der nachfolgenden Tabelle 2 angeführten Daten.

**Tabelle 2: Abbau von β-Carotin, Reaktion Pseudo 1. Ordnung**

| Photoaktivator | K [min⁻¹] | t_{1/2} [min] | Absorptionsmaximum (t = 0 min) [a.u.] | Absorptionsmaximum (t = 5 min) [a.u.] | Abbau [%] |
|---|---|---|---|---|---|
| - | 0,027 | 29 | 1,00 | 0,87 | 13 |
| M1 | 0,31 | 2 | 1,00 | 0,17 | 83 |
| M2 | 0,31 | 2 | 1,00 | 0,20 | 80 |
| M3 | 0,31 | 2 | 1,00 | 0,22 | 78 |
| M4 | 0,31 | 2 | 1,00 | 0,27 | 73 |

## Patentansprüche

1. Verwendung von Alkyloxy-substituierten Anthracen-9,10-dionen der allgemeinen Formel (I), 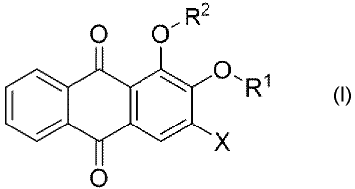 in der R¹ und R² unabhängig voneinander für H, SO₃⁻M⁺ oder einen gegebenenfalls substituierten linearen oder verzweigten Alkylrest mit 1 bis 20 C-Atomen stehen mit der Maßgabe, dass mindestens einer der Reste R¹ oder R² ein gegebenenfalls substituierter linearer oder verzweigter Alkylrest mit 1 bis 20 C-Atomen ist, X für H oder SO₃⁻M⁺ und M⁺ für ein Proton, ein Alkalimetallion oder ein Ammoniumion oder deren Mischungen stehen, als Photobleichmittel zur Entfernung von Anschmutzungen von textilen Materialien.

2. Waschmittel, enthaltend Alkyloxy-substituiertes Anthracen-9,10-dion der allgemeinen Formel (I), 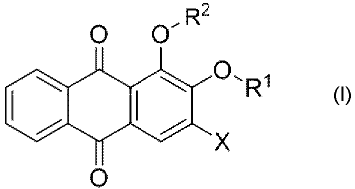 in der R¹ und R² unabhängig voneinander für H, SO₃⁻M⁺ oder einen gegebenenfalls substituierten linearen oder verzweigten Alkylrest mit 1 bis 20 C-Atomen stehen mit der Maßgabe, dass mindestens einer der Reste R¹ oder R² ein gegebenenfalls substituierter linearer oder verzweigter Alkylrest mit 1 bis 20 C-Atomen ist, X für H oder SO₃⁻M⁺ und M⁺ für ein Proton, ein Alkalimetallion oder ein Ammoniumion oder deren Mischungen stehen und ein nichtionisches, anionisches, kationisches und/oder amphoteres Tensid.

3. Mittel nach Anspruch 2, **dadurch gekennzeichnet, dass** es 0,00001 Gew.-% bis 1 Gew.-%, insbesondere 0,001 Gew.-% bis 0,1 Gew.-% Alkyloxy-substituiertes Anthracen-9,10-dion der allgemeinen Formel (I) enthält.

4. Mittel nach Anspruch 2 oder 3, **dadurch gekennzeichnet, dass** es flüssig ist.

5. Mittel nach einem der Ansprüche 2 bis 4, **dadurch gekennzeichnet, dass** es keine Bleichmittel in Form von Hypochloriten oder Persauerstoffverbindungen enthält.

6. Mittel nach einem der Ansprüche 2 bis 5, **dadurch gekennzeichnet, dass** es als vorgefertigte Dosiereinheit mit wasserlöslicher Umhüllung vorliegt.

7. Verfahren zum Entfernen von Anschmutzungen von Textilmaterialien unter Einsatz von Alkyloxy-substituierten Anthracen-9,10-dionen der allgemeinen Formel (I), 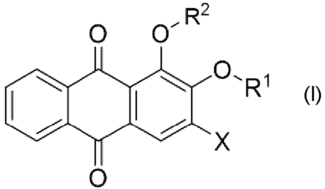 in der R¹ und R² unabhängig voneinander für H, SO₃⁻M⁺ oder einen gegebenenfalls substituierten linearen oder verzweigten Alkylrest mit 1 bis 20 C-Atomen stehen mit der Maßgabe, dass mindestens einer der Reste R¹ oder R² ein gegebenenfalls substituierter linearer oder verzweigter Alkylrest mit 1 bis 20 C-Atomen ist, X für H oder SO₃⁻M⁺ und M⁺ für ein Proton, ein Alkalimetallion oder ein Ammoniumion oder deren Mischungen steht, in einer wässrigen Waschflüssigkeit, in welcher sich die waschbedürftigen angeschmutzten Textilmaterialien befinden, unter Bestrahlung der wässrigen Waschflüssigkeit mit sichtbarem Licht.

8. Verfahren nach Anspruch 7, **dadurch gekennzeichnet, dass** Licht im Wellenlängenbereich von 400 nm bis 600 nm, insbesondere von 450 nm bis 525 nm, zum Einsatz kommt.

9. Verwendung nach Anspruch 1 oder Verfahren nach Anspruch 7 oder 8, **dadurch gekennzeichnet, dass** man das Alkyloxy-substituierte Anthracen-9,10-dion der allgemeinen Formel (I) in Konzentrationen im Bereich von 0,0001 mmol/I bis 0,1 mmol/l, insbesondere von 0,001mmol/l bis 0,01 mmol/I in wässrigen Waschlaugen einsetzt.

10. Verwendung, Mittel oder Verfahren nach einem der voranstehenden Ansprüche, **dadurch gekennzeichnet, dass** in Formel (I) R¹ und R² unabhängig voneinander für einen gegebenenfalls substituierten linearen oder verzweigten Alkylrest mit 1 bis 20 C-Atomen stehen, und/oder dass der Rest R¹ und/oder R² substituiert ist und Substituenten ausgewählt aus -OH, -COO⁻M⁺, -SO₃⁻M⁺, -OSO₃⁻M⁺,-N⁺(CH₂CH₃)₃Hal⁻ und deren Mischungen trägt, wobei M⁺ für ein Proton, ein Alkalimetallion, ein Ammoniumion und deren Mischungen und Hal⁻ für Cl⁻, Br⁻, J⁻, F⁻ und deren Mischungen steht und M⁺ und Hal⁻ auch fehlen können, wenn -COO⁻, -SO₃⁻, -OSO₃⁻ und -N⁺(CH₂CH₃)₃ in ladungsausgleichender Menge vorhanden sind.

## Claims

1. Use of alkyloxy-substituted anthracene-9,10-diones of the general formula (I), 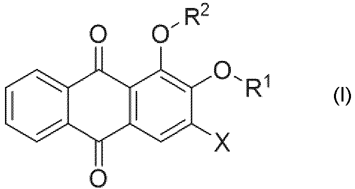 in which R¹ and R² independently of one another represent H, SO₃⁻ M⁺ or an optionally substituted linear or branched alkyl radical having 1 to 20 carbon atoms, with the proviso that at least one of the radicals R¹ or R² is an optionally substituted linear or branched alkyl radical having 1 to 20 carbon atoms, X is H or SO₃⁻ M⁺ and M⁺ is a proton, an alkali metal ion or an ammonium ion or mixtures thereof, as a photo-bleaching agent for removing soiling from textile materials.

2. Detergent containing alkyloxy-substituted anthracene-9,10-dione of the general formula (I), 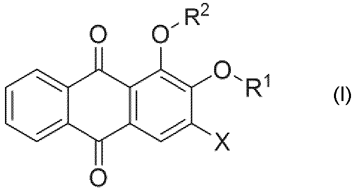 in which R¹ and R² independently of one another represent H, SO₃⁻M⁺ or an optionally substituted linear or branched alkyl radical having 1 to 20 carbon atoms, with the proviso that at least one of the radicals R¹ or R(2) dis an optionally substituted linear or branched alkyl radical having 1 to 20 carbon atoms, X is H or SO₃⁻M⁺ and M⁺ is a proton, an alkali metal ion or an ammonium ion or mixtures thereof, and a nonionic, anionic, cationic and/or amphoteric surfactant.

3. Agent according to claim 2, **characterized in that** it contains 0.00001 wt% to 1 wt%, in particular 0.001 wt% to 0.1 wt%, of alkyloxy-substituted anthracene-9,10-dione of the general formula (I).

4. Agent according to claim 2 or 3, **characterized in that** it is liquid.

5. Agent according to any of claims 2 to 4, **characterized in that** it does not contain bleaching agents in the form of hypochlorites or peroxygen compounds.

6. Agent according to any of claims 2 to 5, **characterized in that** it is available as a prefabricated dosing unit with a water-soluble coating.

7. Method for removing soiling from textile materials using alkyloxy-substituted anthracene-9,10-diones of the general formula (I), 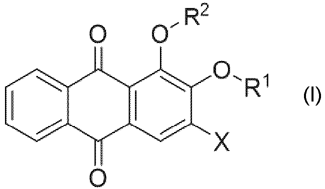 in which R¹ and R² independently of one another represent H, SO₃⁻M⁺ or an optionally substituted linear or branched alkyl radical with 1 to 20 carbon atoms, with the proviso that at least one of the radicals R¹ or R² is an optionally substituted linear or branched alkyl radical having 1 to 20 carbon atoms, X is H or SO₃⁻M⁺ and M⁺ represent a proton, an alkali metal ion or an ammonium ion or mixtures thereof, in an aqueous washing liquid in which the soiled textile materials requiring washing are present, while irradiating the aqueous washing liquid with visible light.

8. Method according to claim 7, **characterized in that** light in the wavelength range from 400 nm to 600 nm, in particular from 450 nm to 525 nm, is used.

9. Use according to claim 1 or process according to claim 7 or 8, **characterized in that** the alkyloxy-substituted anthracene-9,10-dione of the general formula (I) is used in concentrations in the range from 0.0001 mmol/I to 0.1 mmol/l, in particular from 0.001 mmol/I to 0.01 mmol/l, in aqueous washing lyes.

10. Use, agent or process according to one of the preceding claims, **characterized in that** in formula (I) R¹ and R² independently of one another represent a linear or branched alkyl radical with 1 to 20 carbon atoms, which may be substituted, and/or that the radical R¹ and/or R² is substituted and substituents are selected from -OH, -COO⁻ M⁺ , -SO₃⁻M⁺, -OSO₃⁻M⁺, -N⁺(CH₂CH₃)₃Hal⁻ and mixtures thereof, wherein M⁺ represent a proton, an alkali metal ion, an ammonium ion and mixtures thereof, and Hal⁻ represents Cl⁻, Br ,J⁻, F⁻ and mixtures thereof, and M⁺ and Hal⁻ may also be absent if -COO⁻ ,-SO₃-, -OSO₃- and -N⁺(CH₂CH₃)₃ are present in charge-balancing amounts.

## Revendications

1. Utilisation d'anthracène-9,10-diones substituées par un groupe alkyloxy, de formule générale (I), 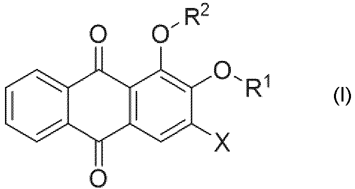 dans laquelle R¹ et R² représentent indépendamment l'un de l'autre H, SO₃⁻M⁺ ou un radical alkyle linéaire ou ramifié éventuellement substitué comportant 1 à 20 atomes de carbone, à condition qu'au moins l'un des radicaux R¹ ou R² représente un radical alkyle linéaire ou ramifié éventuellement substitué comportant 1 à 20 atomes de carbone, X représente H ou SO₃⁻M⁺ et M⁺ représente un proton, un ion de métal alcalin ou un ion ammonium ou leurs mélanges, comme agent de photoblanchiment pour éliminer les salissures des matières textiles.

2. Détergent contenant de l'anthracène-9,10-dione substituée par un groupe alkyloxy, de formule générale (I), 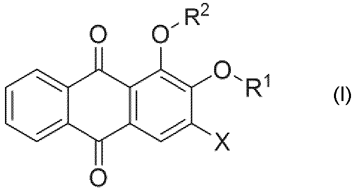 dans laquelle R¹ et R² représentent indépendamment l'un de l'autre H, SO₃⁻M⁺ ou un radical alkyle linéaire ou ramifié éventuellement substitué comportant 1 à 20 atomes de carbone, à condition qu'au moins l'un des radicaux R¹ou R(2) représente un radical alkyle linéaire ou ramifié éventuellement substitué comportant 1 à 20 atomes de carbone, X représente H ou SO₃⁻M⁺ et M⁺ représente un proton, un ion de métal alcalin ou un ion ammonium ou leurs mélanges, et un tensioactif non ionique, anionique, cationique et/ou amphotère.

3. Agent selon la revendication 2, **caractérisé en ce qu'**il contient 0,00001 % en poids à 1 % en poids, en particulier 0,001 % en poids à 0,1 % en poids d'anthracène-9,10-dione substituée par un groupe alkyloxy, de formule générale (I).

4. Agent selon la revendication 2 ou 3, **caractérisé en ce qu'**il est liquide.

5. Agent selon l'une des revendications 2 à 4, **caractérisé en ce qu'**il ne contient pas d'agents blanchissants sous forme d'hypochlorites ou de composés peroxygénés.

6. Agent selon l'une des revendications 2 à 5, **caractérisé en ce qu'**il se présente sous forme d'unité de dosage préfabriquée avec un enrobage soluble dans l'eau.

7. Procédé pour éliminer les salissures des matières textiles à l'aide d'anthracène-9,10-diones substituées par des groupes alkyloxy, de formule générale (I), 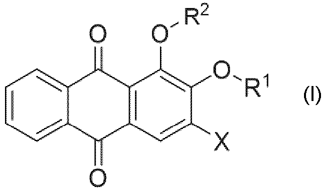 dans laquelle R¹ et R² représentent indépendamment l'un de l'autre H, SO₃⁻M⁺ ou un radical alkyle linéaire ou ramifié éventuellement substitué comportant 1 à 20 atomes de carbone, à condition qu'au moins l'un des radicaux R¹ ou R² représente un radical alkyle linéaire ou ramifié éventuellement substitué comportant 1 à 20 atomes de carbone, X représente H ou SO₃⁻M⁺ et M⁺ représentent un proton, un ion de métal alcalin ou un ion ammonium ou leurs mélanges, dans un liquide de lavage aqueux dans lequel se trouvent les matières textiles souillées nécessitant un lavage, sous irradiation du liquide de lavage aqueux par de la lumière visible.

8. Procédé selon la revendication 7, **caractérisé en ce que** l'on utilise une lumière dans la gamme de longueurs d'onde de 400 nm à 600 nm, en particulier de 450 nm à 525 nm.

9. Utilisation selon la revendication 1 ou procédé selon la revendication 7 ou 8, **caractérisés en ce que** l'on utilise l'anthracène-9,10-dione substituée par un groupe alkyloxy de formule générale (I) en concentrations comprises entre 0,0001 mmol/l et 0,1 mmol/l, en particulier entre 0,001 mmol/l et 0,01 mmol/l, dans des lessives aqueuses.

10. Utilisation, agent ou procédé selon l'une des revendications précédentes, **caractérisé en ce que** dans la formule (I), R¹ et R² représentent indépendamment l'un de l'autre un radical alkyle linéaire ou ramifié éventuellement substitué comportant 1 à 20 atomes de carbone, et/ou **en ce que** le radical R¹ et/ou R² est substitué et les substituants sont choisis parmi -OH, -COO⁻M⁺, -SO₃⁻M⁺, -OSO₃⁻M⁺,-N⁺(CH₂CH)₃Hal⁻ et leurs mélanges, M⁺ représentant un proton, un ion de métal alcalin, un ion ammonium et leurs mélanges et Hal⁻ représentant Cl⁻, Br⁻, J⁻, F⁻ et leurs mélanges, et M⁺ et Hal⁻ pouvant également être absents lorsque -COO⁻, -SO₃⁻ , -OSO₃⁻ et -N⁺(CH₂CH₃)₃ sont présents en quantité compensant la charge.
